# EUROPÄISCHE PATENTSCHRIFT

(11) **EP 2 547 344 B1**
(45) Veröffentlichungstag und Bekanntmachung des Hinweises auf die Patenterteilung: **02.05.2018**
(21) Anmeldenummer: 11709647.9
(22) Anmeldetag: 18.03.2011
(51) Int. Cl.: A61K 31/715, A61P 7/08, A61M 1/28

(54) **VERESTERTE POLYSACCHARID-OSMOTIKA**
ESTERIFIED POLYSACCHARIDE OSMOTIC AGENTS
AGENTS OSMOTIQUES À BASE DE POLYSACCHARIDE ESTÉRIFIÉ

(30) Priorität: 19.03.2010 DE 102010012183
(43) Veröffentlichungstag der Anmeldung: 23.01.2013
(73) Patentinhaber: Fresenius Medical Care Deutschland GmbH, 61352 Bad Homburg (DE)
(72) Erfinder: FICHERT, Thomas, 48231 Warendorf (DE); BICHLMAIER, Ingo, 61206 Wöllstadt (DE); FENN, Dominik, 67657 Kaiserslautern (DE); SCHWEITZER, Thomas, 66589 Wemmetsweiler (DE)
(74) Vertreter: Herrmann, Uwe
(86) Internationale Anmeldenummer: PCT/EP2011/001358
(87) Internationale Veröffentlichungsnummer: WO 2011/113608

(56) Entgegenhaltungen:
- EP-A1- 1 939 219
- EP-A2- 0 602 585
- WO-A1-00/01394
- WO-A1-00/33851
- WO-A1-99/47093
- WO-A2-2006/134492
- BE-A1- 831 179
- DE-A1- 4 123 000
- DE-A1- 4 123 001
- US-A- 2 461 139
- MASSICOTTE L P ET AL: "Carboxylated high amylose starch as pharmaceutical excipients", INTERNATIONAL JOURNAL OF PHARMACEUTICS, ELSEVIER BV, NL, Bd. 356, Nr. 1-2, 22. Mai 2008 (2008-05-22), Seiten 212-223, XP022625191, ISSN: 0378-5173, DOI: DOI:10.1016/J.IJPHARM.2008.01.039 [gefunden am 2008-01-31]
- WOLF BRYAN W ET AL: "Glycemic response to a food starch esterified by 1-octenyl succinic anhydride in humans", JOURNAL OF AGRICULTURAL AND FOOD CHEMISTRY, Bd. 49, Nr. 5, Mai 2001 (2001-05), Seiten 2674-2678, XP002635081, ISSN: 0021-8561
- BAYDOUN L ET AL: "New surface-active polymers for ophthalmic formulations: evaluation of ocular tolerance", EUROPEAN JOURNAL OF PHARMACEUTICS AND BIOPHARMACEUTICS, ELSEVIER SCIENCE PUBLISHERS B.V., AMSTERDAM, NL, Bd. 58, Nr. 1, 1. Juli 2004 (2004-07-01), Seiten 169-175, XP004519841, ISSN: 0939-6411, DOI: DOI:10.1016/J.EJPB.2004.03.005
- BAYDOUN L ET AL: "Influence of n-octenylsuccinate starch on in vitro permeation of sodium diclofenac across excised porcine cornea in comparison to Voltaren ophtha", EUROPEAN JOURNAL OF PHARMACEUTICS AND BIOPHARMACEUTICS, ELSEVIER SCIENCE PUBLISHERS B.V., AMSTERDAM, NL, Bd. 56, Nr. 1, 1. Juli 2003 (2003-07-01), Seiten 73-79, XP004434922, ISSN: 0939-6411, DOI: DOI:10.1016/S0939-6411(03)00036-5
- QU Z H ET AL: "STARCH-BASED INGREDIENTS FOR FLAVOR ENCAPSULATION", CEREAL FOODS WORLD, AMERICAN ASSOCIATION OF CEREAL CHEMISTS, INC, US, Bd. 44, Nr. 7, 1. Juli 1999 (1999-07-01), Seiten 460-465, XP009005508, ISSN: 0146-6283
- Ruth K Mackenzie ET AL: "Bicarbonate/Lactate- and Bicarbonate-Buffered Peritoneal Dialysis Fluids Improve Ex Vivo Peritoneal Macrophage TNFa Secretion", Received November, 1 January 1997 (1997-01-01), XP055377176, Retrieved from the Internet: URL:http://jasn.asnjournals.org/content/9/ 8/1499.full.pdf [retrieved on 2017-05-30]

## Beschreibung

Die vorliegende Erfindung betrifft Dialyselösungen mit veresterten Polysaccharidosmotika.

Osmotisch aktive Verbindungen (Osmotika) finden in der Pharmazie und Medizin breite Anwendung. So werden Osmotika beispielsweise zur Einstellung der Tonizität von Arzneimitteln, insbesondere parenteralen Medikamenten verwendet. Hierbei wird der osmotische Druck eines Arzneimittels je nach Art der Anwendung hypotonisch, hypertonisch oder isotonisch eingestellt. Beispielsweise kann der osmotische Druck einer parenteralen Arzneilösung dem osmotischen Druck des menschlichen Bluts durch Zugabe eines Osmotikums angeglichen werden (isoosmotische Lösungen).

Ferner werden Osmotika bei der Dialysebehandlung, insbesondere bei der Peritonealdialyse eingesetzt, um dem Dialysepatienten überschüssiges Wasser zu entziehen.

Das Peritonealdialyseverfahren beruht darauf, dass eine Lösung, die osmotisch aktive Verbindungen enthält, über einen Katheter in die Bauchhöhle des Dialysepatienten eingebracht wird. Diese Lösung wird für eine bestimmte Zeit (üblicherweise einige Stunden) in der Bauchhöhle des Patienten belassen und entfaltet dort ihre osmotische Wirkung; d.h. dem Patienten wird körpereigenes Wasser in die Bauchhöhle entzogen. Nach einer bestimmten Verweildauer wird die nunmehr verdünnte Peritonealdialyselösung über einen Katheter abgelassen.

Dieses Prinzip findet in verschiedenen Verfahren der Peritonealdialysebehandlung Anwendung. Nach Bedarf können beispielsweise die Verfahren der intermittierenden (IPD), nächtlichen intermittierenden (NIPD), kontinuierlichen zyklischen (CCPD) oder kontinuierlichen ambulanten Peritonealdialyse (CAPD), angewendet werden. Bei IPD, NIPD und CCPD kommen Geräte zum Einsatz, die den Patienten bei der Durchführung des Peritonealdialyseverfahrens unterstützen. Die CAPD ist ein manuelles Verfahren.

Durch die Zugabe von osmotisch aktiven Verbindungen soll insbesondere gewährleistet werden, dass der osmotische Druck der Peritonealdialyselösung während der gesamten Verweildauer in der Bauchhöhle hoch genug ist, um dem Patienten Wasser zu entziehen; d.h. Wasser geht aus dem Kreislauf des Patienten in die Bauchhöhle über (Ultrafiltration).

Jedoch kommt es aufgrund des Wasserübertritts in die Bauchhöhle zwangsläufig zu einer Verdünnung der eingebrachten Peritonealdialyselösung. Diese Verdünnung hat zur Folge, dass die Konzentration der osmotisch aktiven Verbindung und somit auch der osmotische Druck dieser Lösung abnimmt.

Nimmt der osmotische Druck der Peritonealdialyselösung aufgrund dieser Verdünnung ab, so hat dies wiederum zur Folge, dass auch der pro Zeiteinheit stattfindende Wasserübertritt in die Bauchhöhle abnimmt oder möglicherweise gänzlich zum Erliegen kommt. In diesen Fällen findet also mit fortschreitender Verweildauer der Peritonealdialyselösung in der Bauchhöhle des Patienten kein effektiver Wasserentzug mehr statt.

Durch Absorption von osmotisch aktiven Verbindungen in den Blutkreislauf des Patienten, kann sich die Richtung des Wasserübertritts sogar umkehren, d.h. Wasser geht aus der Bauchhöhle in den Blutkreislauf des Patienten über (negative Ultrafiltration). Dies ist dann der Fall, wenn die verdünnte Peritonealdialyselösung in der Bauchhöhle einen geringeren osmotischen Druck als das körpereigene Wasser (z.B. das Blut) des Patienten aufweist.

Durch Zugabe geeigneter osmotisch aktiver Verbindungen zur Peritonealdialyselösung kann der osmotische Druck über eine für die Peritonealdialyse geeignete Behandlungsdauer aufrechterhalten werden, so dass es innerhalb der Verweildauer der Lösung in der Bauchhöhle zu keinem übermäßigen Rückgang der Ultrafiltration kommt. Somit wird auch eine negative Ultrafiltration weitestgehend verhindert.

Die in der Peritonealdialysebehandlung eingesetzten Lösungen enthalten in der Regel Zuckermonomere oder -polymere, wie beispielsweise Glucose oder Polyglucose (z.B. Stärkederivate), als osmotisch wirksame Verbindungen.

EP-B1-0602585 schlägt die Verwendung von Hydroxyethylstärke als Osmotikum vor.

EP-B1-0083360, EP-B2-0115911, EP-B1-0153164 und EP-B1-0207676 betreffen Lösungen für die Peritonealdialyse, die als osmotische aktive Verbindungen Stärkehydrolysat-Glucosepolymere enthalten.

Die DE 41 23 001 A1 und die DE 41 23 000 A1 offenbaren Dialyselösungen mit veresterten Polysaccharidosmotika.

Aufgabe der vorliegenden Erfindung ist, eine Dialyselösung zur Verfügung zu stellen, die Osmotika mit einer höheren osmotischen Wirksamkeit als herkömmliche osmotisch wirksame Verbindungen aufweisen und sich dadurch insbesondere für die Verwendung in der Peritonealdialysebehandlung eignen.

Diese Aufgabe wird durch den Gegenstand der Patentansprüche gelöst.

Die in den erfindungsgemäßen Lösungen enthaltenen Osmotika zeichnen sich gegenüber herkömmlichen Osmotika dadurch aus, dass sie bei gleicher Konzentration eine erhöhte osmotische Wirksamkeit aufweisen.

Diese erhöhte osmotische Wirksamkeit führt insbesondere dazu, dass der Wasserentzug (Ultrafiltration) während der Dialysebehandlung erhöht ist und/oder über einen längeren Zeitraum aufrechterhalten wird. Somit kommt es bei der Verwendung der erfindungsgemäßen Osmotika insbesondere zu einem effektiveren Wasserentzug bei der Dialysebehandlung. Dies kann beispielsweise zur Verkürzung der Dialysebehandlungsdauer beitragen. Alternativ kann die Konzentration an erfindungsgemäßen Osmotikum verringert werden, um die gleiche osmotische Wirksamkeit eines herkömmlichen Osmotikums zu erreichen.

Die Verkürzung der Dialysebehandlungsdauer und/oder die Absenkung der Konzentration kann wiederum zu einer geringeren Inzidenz an unerwünschten Wirkungen beim Dialysepatienten führen.

Durch die Veresterung des Polysaccharids mit der Dicarbonsäure Maleinsäure werden deprotonierbare und somit auch anionisch geladene Seitenketten in das Polysaccharid eingeführt.

Es wurde gefunden, dass durch die Einführung dieser deprotonierbaren bzw. anionischen Seitenketten, die Effizienz der Peritonealdialysebehandlung durch erhöhte Ultrafiltration verbessert wird.

Zum Zwecke dieser Beschreibung umfasst der Begriff "Polysaccharid" Verbindungen, die mindestens zehn Monosaccharid-Monomere enthalten (Pure & Applied Chemistry, 1995, 67, 1360).

Im Sinne dieser Beschreibung umfassen die Begriffe "verestert" und "Ester" Verbindungen, die die Struktureinheit -C(=O)-O- aufweisen (Pure & Applied Chemistry, 1995, 67, 1334).

Maleinsäure ist eine physiologische Dicarbonsäure.

Im Sinne dieser Beschreibung umfasst der Begriff "physiologische Dicarbonsäure" Dicarbonsäuren, die im menschlichen Metabolismus vorkommen.

Im Sinne dieser Beschreibung steht der Begriff "Dicarbonsäure" für eine organische Verbindung, die zwei Säuregruppen (Carboxylgruppen, -COOH) aufweist. Die Säuregruppen können ungeladen, d.h. als -COOH (Carboxyl) oder anionisch, d.h. deprotoniert als -COO⁻(Carboxylat) vorliegen.

Im Fall, dass die Säuregruppe als anionisches Carboxylat vorliegt, kann diese mit einem kationischen Gegenion (z.B. Natrium-, Kalium-, Calcium-, Magnesium-Kation) ein Salz bilden.

Das mittlere Molekulargewicht der erfindungsgemäß verwendeten abgebauten Stärke beträgt 2000 bis 30000 g/mol, bevorzugter 2500 bis 26000 g/mol, noch bevorzugter 3000 bis 22000 g/mol, noch bevorzugter 3500 bis 20000 g/mol, am bevorzugtesten 4000 bis 18000 g/mol und insbesondere 5000 bis 15000 g/mol.

In einer weiteren bevorzugten Ausführungsform beträgt das mittlere Molekulargewicht der erfindungsgemäß verwendeten abgebauten Stärke 15000 bis 25000 g/mol, insbesondere 18000 bis 22000 g/mol.

Die erfindungsgemäß verwendete abgebaute Stärke weist vorzugsweise einen durchschnittlichen Polymerisationsgrad von 10 bis 170, bevorzugter von 11 bis 130, noch bevorzugter von 12 bis 100, am Bevorzugtesten von 13 bis 80 und insbesondere von 14 bis 50 auf.

In einer weiteren bevorzugten Ausführungsform ist der durchschnittliche Polymerisationsgrad der erfindungsgemäß verwendeten abgebauten Stärke 80 bis 140, bevorzugter 85 bis 135, noch bevorzugter 90 bis 130, am Bevorzugtesten 95 bis 125 und insbesondere 100 bis 120.

Vorzugsweise weist eine 7,5 gewichtsprozentige wässrige Lösung der erfindungsgemäß verwendeten abgebauten Stärke eine theoretische Osmolarität >11,9 mosm/L, bevorzugter größer als ≥12,5 mosm/L, noch bevorzugter größer als ≥13,0 mosm/L, am Bevorzugtesten größer als ≥13,5 mosm/L und insbesondere größer als ≥14,0 mosm/L auf.

Zum Zwecke dieser Beschreibung steht der Ausdruck "theoretische Osmolarität" für die theoretisch berechnete Osmolarität. Methoden zur Berechnung dieses Werts sind dem Fachmann bekannt.

In einer bevorzugten Ausführungsform beträgt der kolloidosmotische Druck einer 7,5 gewichtsprozentigen Lösung der erfindungsgemäß verwendeten abgebauten Stärke ≥50 mosm/L oder ≥60 mosm/L, bevorzugter ≥70 mosm/L oder ≥80 mosm/L, noch bevorzugter ≥90 mosm/L oder ≥100 mosm/L, am Bevorzugtesten ≥110 mosm/L oder ≥120 mosm/L und insbesondere ≥130 mosm/L oder ≥140 mosm/L.

In einer weiteren bevorzugten Ausführungsform beträgt der kolloidosmotische Druck einer 7,5 gewichtsprozentigen Lösung der erfindungsgemäß verwendeten abgebauten Stärke ≥150 mosm/L oder ≥160 mosm/L, bevorzugter ≥170 mosm/L oder ≥180 mosm/L, noch bevorzugter ≥190 mosm/L oder ≥200 mosm/L, am Bevorzugtesten ≥210 mosm/L oder ≥220 mosm/L und insbesondere ≥230 mosm/L oder ≥240 mosm/L.

In einer weiteren bevorzugten Ausführungsform beträgt der kolloidosmotische Druck einer 7,5 gewichtsprozentigen Lösung der erfindungsgemäß verwendeten abgebauten Stärke 50 bis 500 mosm/L, bevorzugter 75 mosm/L bis 400 mosm/L, noch bevorzugter 100 bis 300 mosm/L, am Bevorzugtesten 110 mosm/L bis 275 mosm/L und insbesondere 120 mosm/L bis 250 mosm/L.

In einer weiteren bevorzugten Ausführungsform beträgt der kolloidosmotische Druck einer 7,5 gewichtsprozentigen Lösung der erfindungsgemäß verwendeten abgebauten Stärke 100 bis 500 mosm/L, bevorzugter 100 mosm/L bis 400 mosm/L, noch bevorzugter 100 bis 350 mosm/L, am Bevorzugtesten 100 mosm/L bis 325 mosm/L und insbesondere 100 mosm/L bis 290 mosm/L.

Zum Zwecke dieser Beschreibung steht der Ausdruck "kolloidosmotischer Druck" für den experimentell gemessenen osmotischen Druck der Lösung, der sich aus dem osmotischen und onkotischen Druck zusammensetzt. Geeignete Methoden zur experimentellen Bestimmung dieses Werts sind dem Fachmann bekannt.

Die Osmolalität einer 7,5 gewichtsprozentigen wässrigen Lösung der erfindungsgemäß verwendeten abgebauten Stärke beträgt vorzugsweise >16 mosm/kg, bevorzugter ≥18 mosm/kg, noch bevorzugter ≥20 mosm/kg, am Bevorzugtesten ≥22 mosm/kg und insbesondere ≥25 mosm/kg.

Zum Zwecke dieser Beschreibung steht der Begriff "Osmolalität" für die mittels Gefrierpunktserniedrigung experimentell bestimmten Osmolalität der Lösung. Methoden zur Bestimmung der Gefrierpunktserniedrigung sind dem Fachmann bekannt.

Die mittels Gefrierpunkterniedrigung experimentell bestimmte Osmolarität einer 7,5 gewichtsprozentigen wässrigen Lösung der erfindungsgemäß verwendeten abgebauten Stärke beträgt vorzugsweise ≥15 mosm/L, bevorzugter ≥17 mosm/L, noch bevorzugter ≥19 mosm/L, am Bevorzugtesten ≥21 mosm/L und insbesondere ≥23 mosm/L.

Die erfindungsgemäß verwendete abgebaute Stärke ist mit Maleinsäure verestert. Sie weist dabei einen Substitutionsgrad von 0,01 bis 3, bevorzugter von 0,05 bis 2,5, noch bevorzugter von 0,1 bis 2, am Bevorzugtesten von 0,25 bis 1,5 und insbesondere von 0,5 bis 1 auf.

In einer weiteren bevorzugten Ausführungsform weist die erfindungsgemäß verwendete abgebaute Stärke einen Substitutionsgrad von 0,02±0,01 oder 0,05±0,025, bevorzugter von 0,1±0,05, noch bevorzugter von 0,5±0,25, am Bevorzugtesten von 1±0,5 und insbesondere von 1,5±0,75 auf.

In einer besonders bevorzugten Ausführungsform weist die erfindungsgemäß verwendete abgebaute Stärke einen Substitutionsgrad von 0,02±0,005 oder 0,05±0,0125, bevorzugter von 0,1±0,025, noch bevorzugter von 0,5±0,125, am Bevorzugtesten von 1±0,25 und insbesondere von 1,5±0,375 auf.

In einer bevorzugten Ausführungsform handelt es sich bei der erfindungsgemäßen Lösung um eine Lösung zur Verwendung in der Hämo- und/oder Peritonealdialysebehandlung.

Das erfindungsgemäße Polysaccharid ist insbesondere zur Verwendung in der Peritonealdialysebehandlung geeignet.

Die Herstellung des erfindungsgemäß verwendeten Osmotikums kann die Schritte umfassen:
a. Mischen eines Polysaccharids mit einem ersten organischen Lösungsmittel,
b. Mischen der in Schritt a. erhaltenen Dispersion oder Lösung mit einem Maleinsäureanhydrid.

Das in Schritt a. eingesetzte Polysaccharid ist abgebaute Stärke.

In einer bevorzugten Ausführungsform wird zu der in Schritt b. erhaltenen Lösung oder Dispersion ein Katalysator zugesetzt, der die Veresterungsreaktion beschleunigt. Bei diesem Katalysator handelt es sich vorzugsweise um einen nukleophilen Katalysator, vorzugsweise 4-(Dimethylamino)pyridin (DMAP). Andere analog wirkende Katalysatoren sind dem Fachmann bekannt.

Des Weiteren kann auch Base zugesetzt werden, vorzugsweise eine Aminbase wie beispielsweise Triethylamin. Weitere Aminbasen sind dem Fachmann bekannt.

Der Katalysator wird vorzugsweise in katalytischen Mengen zugesetzt; d.h. das Stoffmengenverhältnis von Katalysator (z.B. DMAP) zu Säureanhydrid ist vorzugsweise ≤1:10 oder ≤1:25, bevorzugter ≤1:50 oder ≤1:75, noch bevorzugter ≤1:75 oder ≤1:100, am Bevorzugtesten ≤1:250 und insbesondere ≤1:500.

Nach Schritt b. kann das Reaktionsgemisch bei erhöhter Temperatur gerührt werden. Vorzugsweise beträgt die Temperatur 40 bis 80 °C, bevorzugter 50 bis 70 °C, noch bevorzugter 55 bis 65 °C und insbesondere 60 °C.

In einer bevorzugten Ausführungsform wird das in Schritt b. erhaltene Reaktionsgemisch für 1 bis 12 Stunden, bevorzugter für 2 bis 8 Stunden, noch bevorzugter für 4 bis 6 Stunden und insbesondere für 5 Stunden gerührt.

Das Stoffmengenverhältnis von Säureanhydrid zu Polysaccharid beträgt vorzugsweise 0,1 bis 5 mol/AGU, bevorzugter 0,2 bis 4 mol AGU, noch bevorzugter 0,3 bis 3 mol/AGU, am Bevorzugtesten 0,4 bis 2 mol/AGU und insbesondere 0,5 bis 1 mol/AGU.

In einer weiteren bevorzugten Ausführungsform beträgt das Stoffmengenverhältnis 0,1 bis 2,5 mol/AGU, bevorzugter 0,2 bis 1,75 mol/AGU, noch bevorzugter 0,3 bis 1,5 mol/AGU, am Bevorzugtesten 0,4 bis 1,25 mol/AGU und insbesondere 0,5 bis 0,75 mol/AGU.

Im Sinne dieser Beschreibung steht die Abkürzung "AGU" für "anhydrous glucose unit" (wasserfreie Glucose-Einheit). Dieser Standard-Begriff ist dem Fachmann bekannt.

Das Polysaccharid kann durch Fällung aus der Lösung oder Dispersion abgetrennt werden, wobei das Fällen durch Zugabe eines zweiten organischen Lösungsmittels induziert werden kann.

Die Fällung erfolgt vorzugsweise dadurch, dass das Polysaccharid im ersten organischen Lösungsmittel eine höhere Löslichkeit als im zweiten organischen Lösungsmittel oder der Mischung aus erstem und zweitem organischen Lösungsmittel aufweist.

Das erste organische Lösungsmittel kann jedes beliebige organische Lösungsmittel sein, in dem das Polysaccharid gelöst oder dispergiert werden kann. In einer bevorzugten Ausführungsform ist das erste organische Lösungsmittel Dimethylsulfoxid oder Dimethylacetamid oder eine Mischung davon. Dimethylsulfoxid ist insbesondere bevorzugt.

Das zweite organische Lösungsmittel kann jedes beliebige organische Lösungsmittel sein, in dem das Polysaccharid eine geringere Löslichkeit als im ersten organischen Lösungsmittel aufweist.

Das zweite organische Lösungsmittel ist vorzugsweise ein alkoholisches Lösungsmittel - vorzugsweise Methanol, Ethanol, Propanol, Isopropanol oder Butanol - oder ein Ketonlösungsmittel - vorzugsweise Aceton oder Ethylmethylketon. Als alkoholisches Lösungsmittel ist insbesondere Ethanol bevorzugt. Als Ketonlösungsmittel ist insbesondere Aceton bevorzugt.

Falls Dimethylsulfoxid als erstes organisches Lösungsmittel verwendet wird, ist es insbesondere bevorzugt, dass Ethanol als zweites organisches Lösungsmittel für die Fällung eingesetzt wird.

Falls Dimethylacetamid als erstes organisches Lösungsmittel verwendet wird, ist es insbesondere bevorzugt, dass Aceton als zweites organisches Lösungsmittel für die Fällung eingesetzt wird.

Nach Fällen des Polysaccharids erfolgt die Abtrennung vorzugsweise durch Filtrieren des gefällten Niederschlags.

Der filtrierte Niederschlag wird vorzugsweise getrocknet. Dieser Trocknungsschritt erfolgt vorzugsweise bei erhöhter Temperatur (vorzugsweise 40 °C) und bei erniedrigtem Druck (vorzugsweise Vakuum).

Das Verfahren zur Herstellung der Polysaccharide kann insbesondere die folgenden Schritte umfassen:
- Mischen eines Polysaccharids - nämlich abgebauter Stärke mit Dimethylsulfoxid und/oder Dimethylacetamid,
- Zugeben von Maleinsäureanhydrid, eines nukleophilen Katalysators und ggf. einer Aminbase,
- Rühren des Gemisches bei einer Temperatur von 20 bis 80 °C für 2 bis 12 Stunden,
- Zugeben eines alkoholischen Lösungsmittels oder eines Ketonlösungsmittels, um die Fällung des veresterten Polysaccharids zu induzieren,
- Filtrieren des Niederschlags und
- Trocknen des Niederschlags.

Es kann auch die folgenden Schritte umfassen:
- Mischen eines Polysaccharids mit Dimethylsulfoxid oder Dimethylacetamid,
- Zugeben von Maleinsäureanhydrid, DMAP und ggf. Triethylamin,
- Rühren des Gemisches bei einer Temperatur von 50 bis 70 °C für 3 bis 7 Stunden,
- Zugeben von Ethanol, falls das Lösungsmittel Dimethylsulfoxid ist oder Zugeben von Aceton, falls das Lösungsmittel Dimethylacetamid ist, um die Fällung des erfindungsgemäßen Polysaccharids zu induzieren,
- Filtrieren des Niederschlags und
- Trocknen des Niederschlags.

Gegenstand dieser Erfindung sind Dialyselösungen enthaltend wenigstens ein solchesPolysaccharid.

In einer bevorzugten Ausführungsform ist die erfindungsgemäße Dialyselösung eine Hämodialyselösung oder eine Peritonealdialyselösung. Die erfindungsgemäße Dialyselösung ist insbesondere eine Peritonealdialyselösung.

Darreichungsformen, die in der Dialysebehandlung eingesetzt werden, sind vorzugsweise Konzentrate in Mehrkomponenten-Systemen oder gebrauchsfertige Dialyselösungen.

Für die Zwecke dieser Erfindung umfasst der Ausdruck "Dialyselösung" eine gebrauchsfertige Darreichungsform zur Dialysebehandlung, d.h. eine flüssige Zubereitung, die als solche zur Applikation geeignet ist. Insbesondere muss die Dialyselösung vor der Applikation nicht verdünnt und/ oder mit anderen Zubereitungen gemischt werden.

Im Gegensatz zu den vorstehend beschriebenen Dialyselösungen werden Konzentrate, die entweder in flüssiger, halbfester oder fester Form vorliegen können, vor der Applikation mit Wasser oder wässrigen Lösungen verdünnt oder in Wasser oder wässrigen Lösungen gelöst. In analoger Weise müssen die Komponenten eines Mehrkomponenten-Systems vor der Applikation miteinander gemischt werden um eine gebrauchsfertige Dialyselösung zu erhalten. Konzentrate und Mehrkomponenten-Systeme können also als Vorstufe der erfindungsgemäßen Dialyselösung angesehen werden.

Die erfindungsgemäße Dialyselösung ist vorzugsweise eine Hämodialyse- oder eine Peritonealdialyselösung. Hämodialyse- und Peritonealdialyselösungen enthalten üblicherweise Elektrolyte in einer Konzentration, die im Wesentlichen der Plasma-Elektrolyt-Konzentration entspricht. Elektrolyte umfassen üblicherweise Natrium-, Kalium-, Calcium-, Magnesium- und Chlorid-Ionen.

Dialyselösungen haben üblicherweise einen physiologisch verträglichen pH-Wert. Dies wird vorzugsweise erreicht durch Puffer (Puffer-Systeme), die selbst zum Gesamtgehalt an Elektrolyten beitragen können. Die Puffer sind vorzugsweise Hydrogencarbonat, Lactat oder Pyruvat.

Ferner besitzen Dialyselösungen üblicherweise eine physiologisch verträgliche Osmolarität. Dies wird in der Regel erreicht durch die in der Dialyselösung enthaltenen Elektrolyte und erfindungsgemäßen Polysaccharide, die als osmotisch aktive Verbindungen (Osmotika) in der gewünschten Konzentration physiologisch verträglich sind.

Die erfindungsgemäße Dialyselösung besitzt eine Osmolarität im Bereich von vorzugsweise 200 bis 550 mosm/L.

Im Fall, dass die erfindungsgemäße Dialyselösung eine Hämodialyselösung ist, beträgt die Osmolarität vorzugsweise 200 bis 350 mosm/L oder 210 bis 340 mosm/L, bevorzugter 220 bis 330 mosm/L, noch bevorzugter 230 bis 320 mosm/L, am Bevorzugtesten 240 bis 310 mosm/L und insbesondere 250 bis 300 mosm/L. Verfahren zur Messung der Osmolarität und des osmotischen Drucks sind dem Fachmann bekannt. Beispielsweise können diese mit Hilfe eines Membranosmometers oder anderen geeigneten Messmethoden bestimmt werden.

Im Fall, dass die erfindungsgemäße Dialyselösung eine Peritonealdialyselösung ist, beträgt die Osmolarität vorzugsweise 200 bis 570 mosm/L oder 210 bis 560 mosm/L, bevorzugter 220 bis 550 mosm/L, noch bevorzugter 230 bis 540 mosm/L, am Bevorzugtesten 240 bis 530 mosm/L und insbesondere 250 bis 520 mosm/L. In einer bevorzugten Ausführungsform beträgt die Osmolarität 250 ± 50 mosm/L oder 250 ± 45 mosm/L, bevorzugter 250 ± 35 mosm/L, noch bevorzugter 250 ± 25 mosm/L, am Bevorzugtesten 250 ± 15 mosm/L und insbesondere 250 ± 10 mosm/L. In einer weiteren bevorzugten Ausführungsform beträgt die Osmolarität 300 ± 50 mosm/L oder 300 ± 45 mosm/L, bevorzugter 300 ± 35 mosm/L, noch bevorzugter 300 ± 25 mosm/L, am Bevorzugtesten 300 ± 15 mosm/L und insbesondere 300 ± 10 mosm/L. In einer weiteren bevorzugten Ausführungsform beträgt die Osmolarität 350 ± 50 mosm/L oder 350 ± 45 mosm/L, bevorzugter 350 ± 35 mosm/L, noch bevorzugter 350 ± 25 mosm/L, am Bevorzugtesten 350 ± 15 mosm/L und insbesondere 300 ± 10 mosm/L. In einer weiteren bevorzugten Ausführungsform beträgt die Osmolarität 400 ± 50 mosm/L oder 400 ± 45 mosm/L, bevorzugter 400 ± 35 mosm/L, noch bevorzugter 400 ± 25 mosm/L, am Bevorzugtesten 400 ± 15 mosm/L und insbesondere 300 ± 10 mosm/L. In einer weiteren bevorzugten Ausführungsform beträgt die Osmolarität 450 ± 50 mosm/L oder 450 ± 45 mosm/L, bevorzugter 450 ± 35 mosm/L, noch bevorzugter 450 ± 25 mosm/L, am Bevorzugtesten 450 ± 15 mosm/L und insbesondere 450 ± 10 mosm/L. In einer weiteren bevorzugten Ausführungsform beträgt die Osmolarität 500 ± 50 mosm/L oder 500 ± 45 mosm/L, bevorzugter 500 ± 35 mosm/L, noch bevorzugter 500 ± 25 mosm/L, am Bevorzugtesten 500 ± 15 mosm/L und insbesondere 500 ± 10 mosm/L.

Die erfindungsgemäße Dialyselösung hat einen pH-Wert vorzugsweise von 4,0 bis 8,0, bevorzugter von 4,2 bis 7,5, noch bevorzugter von 4,4 bis 6,8, am Bevorzugtesten von 4,6 bis 6,0 oder 4,8 bis 5,5 und insbesondere von 5,0 bis 5,2 oder 5,0±0,1; gemessen bei Raumtemperatur (20 bis 23 °C). In einer bevorzugten Ausführungsform ist der pH-Wert 4,8 ± 1,0 oder 4,8 ± 0.8, bevorzugter 4,8 ± 0,7 oder 4,8 ± 0,6, noch bevorzugter 4,8 ± 0,5 oder 4,8 ± 0,4, am Bevorzugtesten 4,8 ± 0,3 oder 4,8 ± 0,2 und insbesondere 4,8 ± 0,1. In einer weiteren bevorzugten Ausführungsform ist der pH-Wert 5,0 ± 1,0 oder 5,0 ± 0.8, bevorzugter 5,0 ± 0,7 oder 5,0 ± 0,6, noch bevorzugter 5,0 ± 0,5 oder 5,0 ± 0,4, am Bevorzugtesten 5,0 ± 0,3 oder. 5,0 ± 0,2 und insbesondere 5,0 ± 0,1. In einer weiteren bevorzugten Ausführungsform ist der pH-Wert 5,2 ± 1,0 oder 5,2 ± 0,8, bevorzugter 5,2 ± 0,7 oder 5,2 ± 0,6, noch bevorzugter 5,2 ± 0,5 oder 5,2 ± 0,4, am Bevorzugtesten 5,2 ± 0,3 oder 5,2 ± 0,2 und insbesondere 5,2 ± 0,1. In einer weiteren bevorzugten Ausführungsform ist der pH-Wert 5,5 ± 1,0 oder 5,5 ± 0,8, bevorzugter 5,5 ± 0,7 oder 5,5 ± 0,6, noch bevorzugter 5,5 ± 0,5 oder 5,5 ± 0,4, am Bevorzugtesten 5,5 ± 0,3 oder 5,5 ± 0,2 und insbesondere 5,5 ± 0,1. In einer weiteren bevorzugten Ausführungsform ist der pH-Wert 6,0 ± 1,0 oder 6,0 ± 0.8, bevorzugter 6,0 ± 0,7 oder 6,0 ± 0,6, noch bevorzugter 6,0 ± 0,5 oder 6,0 ± 0,4, am Bevorzugtesten 6,0 ± 0,3 oder 6,0 ± 0,2 und insbesondere 6,0 ± 0,1. In einer weiteren bevorzugten Ausführungsform ist der pH-Wert 6,5 ± 1,0 oder 6,5 ± 0.8, bevorzugter 6,5 ± 0,7 oder 6,5 ± 0,6, noch bevorzugter 6,5 ± 0,5 oder 6,5 ± 0,4, am Bevorzugtesten 6,5 ± 0,3 oder 6,5 ± 0,2 und insbesondere 6,5 ± 0,1. In einer weiteren bevorzugten Ausführungsform ist der pH-Wert 7,0 ± 1,0 oder 7,0 ± 0.8, bevorzugter 7,0 ± 0,7 oder 7,0 ± 0,6, noch bevorzugter 7,0 ± 0,5 oder 7,0 ± 0,4, am Bevorzugtesten 7,0 ± 0,3 oder 7,0 ± 0,2 und insbesondere 7,0 ± 0,1. In einer weiteren bevorzugten Ausführungsform ist der pH-Wert 7,4 ± 1,0 oder 7,4 ± 0.8, bevorzugter 7,4 ± 0,7 oder 7,4 ± 0,6, noch bevorzugter 7,4 ± 0,5 oder 7,4 ± 0,4, am Bevorzugtesten 7,4 ± 0,3 oder 7,4 ± 0,2 und insbesondere 7,4 ± 0,1. In einer weiteren bevorzugten Ausführungsform ist der pH-Wert 8,0 ± 1,0 oder 8,0 ± 0.8, bevorzugter 8,0 ± 0,7 oder 8,0 ± 0,6, noch bevorzugter 8,0 ± 0,5 oder 8,0 ± 0,4, am Bevorzugtesten 8,0 ± 0,3 oder 8,0 ± 0,2 und insbesondere 8,0 ± 0,1.

Die erfindungsgemäße Dialyselösung enthält ein oder mehrere (z.B. zwei, drei, vier oder fünf) erfindungsgemäße Polysaccharide; wobei die erfindungsgemäßen Polysaccharide wie oben stehend definiert sind.

Die erfindungsgemäße Dialyselösung enthält Polysaccharid in einer Gesamtkonzentration von vorzugsweise 0,001 mM bis 10 M oder 0,01 bis 1,0 M, bevorzugter 0,10 bis 500 mM, noch bevorzugter 1,0 bis 250 mM, am Bevorzugtesten 10 bis 100 mM und insbesondere 25 bis 90 mM. In einer bevorzugten Ausführungsform ist die Gesamtkonzentration 25 ± 24 mM, bevorzugter 25 ± 20 mM, noch bevorzugter 25 ± 15 mM, am Bevorzugtesten 25 ± 10 mM und insbesondere 25 ± 5 mM. In einer weiteren bevorzugten Ausführungsform ist die Gesamtkonzentration 50 ± 25 mM, bevorzugter 50 ± 20 mM, noch bevorzugter 50 ± 15 mM, am Bevorzugtesten 50 ± 10 mM und insbesondere 50 ± 5 mM. In einer weiteren bevorzugten Ausführungsform ist die Gesamtkonzentration 75 ± 25 mM, bevorzugter 75 ± 20 mM, noch bevorzugter 75 ± 15 mM, am Bevorzugtesten 75 ± 10 mM und insbesondere 75 ± 5 mM. In einer weiteren bevorzugten Ausführungsform ist die Gesamtkonzentration 100 ± 25 mM, bevorzugter 100 ± 20 mM, noch bevorzugter 100 ± 15 mM, am Bevorzugtesten 100 ± 10 mM und insbesondere 100 ± 5 mM. In einer weiteren bevorzugten Ausführungsform ist die Gesamtkonzentration 200 ± 25 mM, bevorzugter 200 ± 20 mM, noch bevorzugter 200 ± 15 mM, am Bevorzugtesten 200 ± 10 mM und insbesondere 200 ± 5 mM. Die Gesamtkonzentration ist vorzugsweise berechnet mittels des mittleren Molekulargewichts der Polysaccharide.

Die erfindungsgemäße Dialyselösung enthält Polysaccharid in einer Gesamtmassenkonzentration von vorzugsweise 0,01 g/L bis 1,0 kg/L, bevorzugter von 0,1 bis 750 g/L, noch bevorzugter von 1,0 bis 500 g/L, am Bevorzugtesten 10 bis 250 g/L und insbesondere von 100 bis 200 g/L. In einer bevorzugten Ausführungsform ist die Gesamtmassenkonzentration 25 ± 24 g/L, bevorzugter 25 ± 20 g/L, noch bevorzugter 25 ± 15 g/L, am Bevorzugtesten 25 ± 10 g/L und insbesondere 25 ± 5 g/L. In einer weiteren bevorzugten Ausführungsform ist die Gesamtmassenkonzentration 50 ± 25 g/L, bevorzugter 50 ± 20 g/L, noch bevorzugter 50 ± 15 g/L, am Bevorzugtesten 50 ± 10 g/L und insbesondere 50 ± 5 g/L. In einer weiteren bevorzugten Ausführungsform ist die Gesamtmassenkonzentration 75 ± 25 g/L, bevorzugter 75 ± 20 g/L, noch bevorzugter 75 ± 15 g/L, am Bevorzugtesten 75 ± 10 g/L und insbesondere 75 ± 5 g/L. In einer weiteren bevorzugten Ausführungsform ist die Gesamtmassenkonzentration 100 ± 25 g/L, bevorzugter 100 ± 20 g/L, noch bevorzugter 100 ± 15 g/L, am Bevorzugtesten 100 ± 10 g/L und insbesondere 100 ± 5 g/L. In einer weiteren bevorzugten Ausführungsform ist die Gesamtmassenkonzentration 200 ± 25 g/L, bevorzugter 200 ± 20 g/L, noch bevorzugter 200 ± 15 g/L, am Bevorzugtesten 200 ± 10 g/L und insbesondere 200 ± 5 g/L.

Die erfindungsgemäße Dialyselösung kann auch weitere osmotisch aktive Substanzen wie beispielsweise Glucose, Polyglucose, quervernetzte Glucose oder Polyglucose, Mannitol oder Glycerol enthalten.

Die erfindungsgemäße Dialyselösung enthält vorzugsweise einen oder mehrere Elektrolyte.

Im Sinne dieser Erfindung steht der Ausdruck "Elektrolyt" für eine Substanz, die freie Ionen enthält und elektrische Konduktivität aufweist. Vorzugsweise dissoziiert das Elektrolyt vollständig in Kationen und Anionen ohne den pH-Wert einer wässrigen Zusammensetzung im Wesentlichen zu ändern. Diese Eigenschaft grenzt Elektrolyte von Puffersubstanzen ab. Vorzugsweise liegen die Elektrolyte in einer Konzentration vor, die in einer im Wesentlichen vollständigen Dissoziation in Wasser resultiert.

Bevorzugte Elektrolyte sind ausgewählt aus der Gruppe der Alkalimetalle wie beispielsweise Na⁺ und K⁺ und der Erdalkalimetalle wie beispielsweise Ca²⁺ und Mg²⁺. Ein bevorzugtes Anion ist Cl⁻.

Die erfindungsgemäße Dialyselösung kann weitere Anionen wie beispielsweise Hydrogencarbonat, Dihydrogenphosphat, Hydrogenphosphat, Phosphat, Acetat, Lactat und Pyruvat enthalten; diese Anionen (in geeigneten Kombinationen mit Kationen) werden jedoch aufgrund Ihrer Pufferkapazität im Sinne dieser Erfindung nicht als Elektrolyte sondern als Puffer bezeichnet.

In einer bevorzugten Ausführungsform enthält die erfindungsgemäße Dialyselösung Na+-Ionen. Die Konzentration an Na⁺-Ionen ist vorzugsweise 10 bis 200 mM oder 50 bis 190 mM, bevorzugter 100 bis 180 mM oder 110 bis 170 mM, noch bevorzugter 115 bis 165 mM oder 120 bis 160 mM, am Bevorzugtesten 125 bis 155 mM und insbesondere 130 bis 150 mM. In einer weiteren bevorzugten Ausführungsform enthält die erfindungsgemäße Dialyselösung keine Na⁺-Ionen.

In einer bevorzugten Ausführungsform enthält die erfindungsgemäße Dialyselösung K⁺-Ionen. Die Konzentration an K⁺-Ionen ist vorzugsweise 0,10 bis 20 mM, bevorzugter 0,25 bis 15 mM, noch bevorzugter 0,50 bis 10 mM, am Bevorzugtesten 0,75 bis 7,5 mM und insbesondere 1,0 bis 5,0 mM. In einer weiteren bevorzugten Ausführungsform ist die Konzentration an K⁺-Ionen 1,0 ± 0,75, 2,0 ± 0,75, 3,0 ± 0,75, 4,0 ± 0,75 oder 5,0 ± 0,75 mM und insbesondere 1,0 ± 0,50, 2,0 ± 0,50, 3,0 ± 0,50, 4,0 ± 0,50 oder 5,0 ± 0,50. In einer weiteren bevorzugten Ausführungsform enthält die erfindungsgemäße Dialyselösung keine K⁺-Ionen.

In einer bevorzugten Ausführungsform enthält die erfindungsgemäße Dialyselösung Ca²⁺-Ionen. Die Konzentration an Ca²⁺-Ionen ist vorzugsweise 0,1 bis 3 mM, bevorzugter 0,25 bis 2,75 mM, noch bevorzugter 0,5 bis 2,5 mM, am Bevorzugtesten 0,75 bis 2,25 mM und insbesondere 1 bis 2 mM. In einer weiteren bevorzugten Ausführungsform ist die Konzentration an Ca²⁺-Ionen 0,25, 0,5, 0,75, 1, 1,25, 1,5, 1,75 oder 2 mM. In einer weiteren bevorzugten Ausführungsform enthält die erfindungsgemäße Dialyselösung keine Ca²⁺-Ionen.

In einer bevorzugten Ausführungsform enthält die erfindungsgemäße Dialyselösung Mg²⁺-Ionen. Die Konzentration an Mg²⁺-Ionen ist vorzugsweise 0,01 bis 1 mM, bevorzugter 0,05 bis 0,75 mM, noch bevorzugter 0,1 bis 0,5 mM, am Bevorzugtesten 0,15 bis 0,4 mM und insbesondere 0,2 bis 0,3 mM. In einer weiteren bevorzugten Ausführungsform ist die Konzentration an Mg²⁺-Ionen 0,05, 0,075, 0,1, 0,2, 0,25, 0,50 oder 0,75 mM. In einer weiteren bevorzugten Ausführungsform enthält die erfindungsgemäße Dialyselösung keine Mg²⁺-Ionen.

In einer bevorzugten Ausführungsform enthält die erfindungsgemäße Dialyselösung Cl⁻-Ionen. Die Konzentration an Cl⁻-Ionen ist vorzugsweise 10 bis 300 mM, bevorzugter 25 bis 250 mM, noch bevorzugter 50 bis 200 mM, am Bevorzugtesten 75 bis 150 mM und insbesondere 80 bis 125 mM. In einer weiteren bevorzugten Ausführungsform ist die Konzentration an Cl⁻-Ionen 100 ± 50 mM, bevorzugter 100 ± 25 mM, am Bevorzugtesten 100 ± 10 mM und insbesondere 96 ± 4 mM. In einer weiteren bevorzugten Ausführungsform enthält die erfindungsgemäße Dialyselösung keine Cl⁻-Ionen.

Die erfindungsgemäße Dialyselösung enthält vorzugsweise einen oder mehrere Puffer.

Geeignete Puffer sind dem Fachmann bekannt. Üblicherweise umfassen Puffer Lactat-, Hydrogencarbonat-, Carbonat-, Dihydrogenphosphat-, Hydrogenphosphat-, Phosphat-, Pyruvat-, Citrat-, Isocitrat-, Succinat-, Fumarat-, Acetat- und Lactat-Salze. Der Fachmann weiß, dass das entsprechende Kation der vorstehend genannten Anionen Bestandteil des Puffers ist, der zur Einstellung des pH-Wertes benutzt wird (z.B. Na^{⊕} als Bestandteil des Puffers NaHCO₃). Wenn das Puffersalz jedoch in Wasser dissoziiert hat es auch die Wirkung eines Elektrolyts. Für die Zwecke dieser Beschreibung berechnen sich die Konzentrationen an Kationen oder Anionen und die Gesamtkonzentration an Ionen, unabhängig davon, ob sie als Bestandteil von Elektrolyten, Puffern oder anderen Verbindungen (z.B. als Salz der erfindungsgemäßen Polysaccharide) eingesetzt werden.

In einer bevorzugten Ausführungsform enthält der Puffer Hydrogencarbonat. Hydrogencarbonat ist ein gut verträgliches Puffersystem, das im alkalischen Milieu mit Carbonat und im sauren Milieu mit H₂CO₃ bzw. CO₂ im Gleichgewicht steht. Neben Hydrogencarbonat sind auch andere Puffer-Systeme einsetzbar, die im Bereich von pH 4 bis pH 8, bevorzugter im Bereich von pH 5 bis pH 7,6 und insbesondere im Bereich von pH 7,6, 7,4, 7,2 und/oder 7,0 eine Pufferwirkung ausüben; z.B. auch Verbindungen, die im Körper zu Hydrogencarbonat metabolisiert werden können wie Lactat oder Pyruvat.

In einer weiteren bevorzugten Ausführungsform enthält der Puffer das Salz einer schwachen Säure, vorzugsweise Lactat. Die Säurestärke (pKₛ) der schwachen Säure ist vorzugsweise ≤5.

Der Puffer kann auch ein Gemisch von Substanzen mit Pufferwirkung sein, z.B. ein Gemisch enthaltend Hydrogencarbonat und ein Salz einer schwachen Säure (z.B. Lactat). Eine geringe Hydrogencarbonat-Konzentration hat den Vorteil, dass der CO₂-Druck im Behältnis gering ist.

In einer bevorzugten Ausführungsform ist die erfindungsgemäße Dialyselösung gepuffert durch Hydrogencarbonat. Die Hydrogencarbonat-Konzentration ist vorzugsweise 1,0 bis 200 mM, bevorzugter 2,5 bis 150 mM, noch bevorzugter 5 bis 100 mM, am Bevorzugtesten 5 bis 75 mM oder 10 bis 50 mM und insbesondere 20 bis 30 mM. In einer weiteren bevorzugten Ausführungsform ist die Hydrogencarbonat-Konzentration 25 mM. In einer weiteren bevorzugten Ausführungsform enthält die erfindungsgemäße Dialyselösung kein Hydrogencarbonat.

In einer bevorzugten Ausführungsform ist die erfindungsgemäße Dialyselösung gepuffert durch Lactat. Die Lactat-Konzentration ist vorzugsweise 1,0 bis 200 mM, bevorzugter 2,5 bis 150 mM, noch bevorzugter 5 bis 100 mM, am Bevorzugtesten 10 bis 50 mM oder 10 bis 25 mM und insbesondere 15 mM. In einer weiteren bevorzugten Ausführungsform enthält die erfindungsgemäße Dialyselösung kein Lactat.

In einer bevorzugten Ausführungsform ist die erfindungsgemäße Dialyselösung gepuffert durch Acetat. Die Acetat-Konzentration ist vorzugsweise 1,0 bis 100 mM, bevorzugter 1,0 bis 50 mM, noch bevorzugter 1,0 bis 25 mM, am Bevorzugtesten 1,0 bis 10 mM oder 2,0 bis 7,5 mM und insbesondere 2,5 bis 7,0 mM. In einer weiteren bevorzugten Ausführungsform enthält die erfindungsgemäße Dialyselösung kein Acetat.

Das Gesamtvolumen an Dialyselösung ist nicht beschränkt. Üblicherweise beträgt das Volumen mehrere Liter (geeignetes Verabreichungsvolumen für einen Patienten) bis einige hundert Liter (geeignetes Vorratsvolumen für mehr als einen Patienten).

Wie bereits oben stehend ausgeführt ist unter dem Ausdruck "Dialyselösung" im Sinne dieser Erfindung eine gebrauchsfertige Dialyselösung zu verstehen, d.h. die Dialyselösung kann direkt für die Dialysebehandlung (Hämodialyse oder Peritonealdialyse) verwendet werden.

In einer bevorzugten Ausführungsform ist die erfindungsgemäße Dialyselösung eine Peritonealdialyselösung wie nachstehend beschrieben.

Die Peritonealdialyselösung ist biochemisch so abgestimmt, dass sie die mit Nierenversagen einhergehende metabolische Azidose im Wesentlichen korrigiert. Die Peritonealdialyselösung enthält Hydrogencarbonat vorzugsweise in annähernd physiologischen Konzentrationen. In einer bevorzugten Ausführungsform enthält die Peritonealdialyselösung Hydrogencarbonat in einer Konzentration von ca. 20 bis 30 mM. In einer weiteren bevorzugten Ausführungsform enthält die Peritonealdialyselösung eine Hydrogencarbonat-Konzentration von 25 mM.

Ferner enthält die Peritonealdialyselösung vorzugsweise Kohlenstoffdioxid mit einem Partialdruck (pCO₂) von weniger als 60 mmHg. In einer bevorzugten Ausführungsform ist pCO₂ der Peritonealdialyselösung im Wesentlichen gleich zum pCO₂, der in Blutgefäßen gemessen wird.

Ferner hat die Peritonealdialyselösung vorzugsweise einen pH-Wert von ca. 7,4. Daher ist die Peritonealdialyselösung eine physiologisch verträgliche Lösung.

Die Peritonealdialyselösung enthält vorzugsweise eine schwache Säure mit einem pKₛ ≤5. Die schwachen Säuren sind vorzugsweise Verbindungen, die als physiologische Stoffwechselprodukte im Glucose-Metabolismus auftreten. Die schwache Säure ist vorzugsweise ausgewählt aus Gruppe bestehend aus Lactat, Pyruvat, Citrat, Isocitrat, Ketoglutarat, Succinat, Fumarat, Malat und Oxaloacetat. Diese Säuren können entweder allein oder als Gemisch in der Peritonealdialyselösung enthalten sein. Die schwachen Säuren sind vorzugsweise in einer Konzentration von 10 bis 20 mEq/L und im Wesentlichen als NatriumSalze in der Peritonealdialyselösung enthalten. In der Peritonealdialyselösung ist die schwache Säure vorzugsweise in einer Menge enthalten, die der täglichen metabolischen Wasserstoffproduktion von ca. 1 mEq/kg/Tag entspricht.

Die Peritonealdialyselösung enthält wenigstens ein erfindungsgemäßes Polysaccharid wie oben stehend definiert.

Die erfindungsgemäße Peritonealdialyselösung enthält vorzugsweise eine Konzentration an Hydrogencarbonat und weist einen pCO₂ auf, wie sie bei gesunden, nicht-niereninsuffizienten Patienten gemessen werden. Die schwache Säure diffundiert entlang des Konzentrationsgradienten von der Dialyselösung ins Blut des Dialysepatienten und korrigiert somit die metabolische Azidose der Dialysepatienten.

Ein weiterer Gegenstand dieser Erfindung betrifft Mehrkomponenten-Systeme zur Herstellung der oben beschriebenen gebrauchsfertigen Dialyselösungen. Die Herstellung erfolgt vorzugsweise in einer detailliert beschriebenen Art und Weise, d.h. durch Befolgung einer entsprechenden Anleitung (Protokoll). Besagte Herstellung kann manuell, z.B. durch Mischen einzelner Komponenten oder Verdünnen einer Komponente mit Wasser, erfolgen. Die Herstellung kann jedoch auch automatisiert erfolgen, z.B. mittels einer Vorrichtung die für diesen Prozess geeignet ist und kommerziell erhältlich sein kann. Die Herrichtung muss nicht zwangsläufig zu einer Dialyselösung mit statischer (gleich bleibender) Zusammensetzung führen sondern kann auch zu einer Dialyselösung führen, die kontinuierlich ihre Zusammensetzung ändert, wobei diese Änderung durch eine geeignete Vorrichtung überwacht werden kann. Beispielsweise kann das erfindungsgemäße Polysaccharid in einer Dialyselösung enthalten sein, die kontinuierlich während der Dialysebehandlung verdünnt wird, so dass der Patient einer abnehmenden Polysaccharid-Konzentration ausgesetzt ist.

In einer bevorzugten Ausführungsform sind die erfindungsgemäßen Dialyselösungen zur Verwendung in der Behandlung der Niereninsuffizienz geeignet.

Die erfindungsgemäßen Dialyselösungen sind zur Verwendung in der Dialysebehandlung geeignet.

In einer weiteren bevorzugten Ausführungsform sind die erfindungsgemäßen Dialyselösungen zur Verwendung in der Hämodialyse- und / oder Peritonealdialysebehandlung geeignet.

Ein Kit, das für die Herstellung der erfindungsgemäßen Dialyselösungen konfiguriert ist, kann
- eine erste Komponente,
- eine zweite Komponente und
- ggf. eine weitere oder mehrere weitere Komponenten
umfassen, und
die Herstellung der erfindungsgemäßen Dialyselösung durch Mischen der ersten Komponente mit der zweiten Komponente und ggf. der/ den weiteren Komponente(n) erfolgen.

Das Kit umfasst wenigstens eine erste Komponente und eine zweite Komponente. Das Kit kann auch weitere Komponenten umfassen, z.B. eine dritte und eine vierte Komponente. Vorzugsweise besteht das Kit aus zwei Komponenten, die vorzugsweise voneinander verschieden sind.

Im Sinne dieser Erfindung umfasst der Ausdruck "Komponente" flüssige, halbfeste oder feste Zusammensetzungen, die zueinander gleich oder voneinander verschieden sein können, wobei durch Mischen aller Komponenten des Kits die erfindungsgemäße gebrauchsfertige Dialyselösung erhalten wird. Vorzugsweise enthält eine einzelne Komponente einen Teil der Inhaltsstoffe, die in der gebrauchsfertigen Dialyselösung enthalten sind.

Die erste und die zweite Komponente können, unabhängig voneinander, fest, halbfest oder flüssig sein. Im Falle, dass die Komponenten flüssig sind, können sie Lösungen oder Dispersionen (z.B. Dispersionen oder Suspensionen) sein.

In einer bevorzugten Ausführungsform ist die erste Komponente flüssig, vorzugsweise reines Wasser oder eine wässrige Lösung, und die zweite Komponente ist ebenfalls flüssig. In einer weiteren bevorzugten Ausführungsform ist die erste Komponente flüssig, vorzugsweise reines Wasser oder eine wässrige Lösung, und die zweite Komponente ist fest, vorzugsweise ein pulverförmiges Gemisch.

Die erste Komponente ist vorzugsweise eine Lösung, die osmotisch wirksame Substanzen (z.B. Polysaccharid), Calcium-Ionen, Magnesium-Ionen, Hydronium-Ionen und Chlorid-Ionen enthält.

Das Kit kann verschiedenartig ausgestaltet sein. Beispielsweise können die einzelnen Komponenten in voneinander separierten Behältnissen (z.B. einzelne Beutel) vorliegen. Das Kit ist jedoch vorzugsweise ein Gebinde, wie beispielsweise ein Mehrkammer-Behältnis-System (z.B. flexibles oder starres Mehrkammer-Behältnis-System), vorzugsweise ein flexibles Mehrkammer-Beutel-System.

Das Kit ist vorzugsweise ein Mehrkammer-Behältnis-System, das die erste Komponente, die zweite Komponente und ggf. eine oder mehrere weitere Komponenten in Kammern enthält, die voneinander durch lösbare bzw. brechbare Trennsysteme (z.B. Trennbrechteile) getrennt sind, wobei die erste Komponente, die zweite Komponente und ggf. die eine oder mehreren weiteren Komponenten nach dem Lösen bzw. Brechen des Trennsystems miteinander gemischt werden können, um die erfindungsgemäße Dialyselösung zu erhalten.

Das Mehrkammer-Behältnis kann als Kunststoffgebinde vorliegen (z.B. Mehrkammer-Kunststoffbeutel), der jeweils eine abgetrennte Kammer für jede einzelne Komponente enthält. Vorzugsweise enthält das Kunststoffgebinde die einzelnen Komponentenlösungen in Kammern, die jeweils durch Trennelemente voneinander abgetrennt sind.

Das Mehrkammer-Behältnis ist vorzugsweise ein Zweikammer-Beutel umfassend ein Kunststoffgebinde mit einer ersten Kammer und einer zweiten Kammer, wobei die Kammern durch ein lösbares bzw. brechbares Trennsystem voneinander getrennt sind, und die erste Kammer die erste Komponente enthält und die zweite Kammer die zweite Komponente enthält. Das Lösen bzw. Brechen des Trennsystems führt zum Mischen der beiden Komponenten und resultiert in der gebrauchsfertigen Dialyselösung. Die erste Kammer und die zweite Kammer sind im Gebinde vorzugsweise angrenzend angeordnet und durch das Trennsystem voneinander abgetrennt. Das Trennsystem ist vorzugsweise eine Trennnaht (z.B. lösbare oder brechbare Schweißnaht). Die Trennnaht öffnet sich vorzugsweise durch das Anlegen eines Druckes auf eine der Kammern, woraufhin die Trennnaht bricht bzw. sich löst und sich der Inhalt der beiden Kammern mischt und das Gemisch als gebrauchsfertige Dialyselösung in der Dialysebehandlung eingesetzt werden kann.

Die erste Komponente des Kits ist vorzugsweise eine sterile Lösung, die eine Säure enthält und einen pH-Wert ≤ 6,0 aufweist; die zweite Komponente ist vorzugsweise ebenfalls eine sterile Lösung, die vorzugsweise einen Puffer enthält und einen pH-Wert ≥ 7,0 aufweist.

Das Polysaccharid kann in der ersten Komponente oder in der zweiten Komponente als auch in beiden Komponenten in gleichen oder verschiedenen Konzentrationen enthalten sein. In einer bevorzugten Ausführungsform ist das erfindungsgemäße Polysaccharid nur in der ersten (sauren) Komponente enthalten. In einer weiteren bevorzugten Ausführungsform ist das erfindungsgemäße Polysaccharid nur in der zweiten (basischen) Komponente enthalten. Die erste Komponente und/ oder die zweite Komponente und/ oder die ggf. weitere(n) Komponente(n) können einen oder mehrere Elektrolyte aber auch Puffer enthalten.

Der Fachmann erkennt, dass das Mischen der einzelnen Komponenten üblicherweise einen Verdünnungseffekt für den Fall nach sich zieht, dass die Komponenten die Inhaltsstoffe in unterschiedlichen Konzentrationen enthalten. Falls beispielsweise das erfindungsgemäße Polysaccharid ausschließlich in einer der Komponenten enthalten ist, führt das Mischen dieser Komponente mit wenigstens einer anderen Komponente zu einem Anstieg des Volumens in Bezug auf die vorhandene Menge des erfindungsgemäßen Polysaccharids und somit zu einer Verdünnung, d.h. Abnahme der Polysaccharid-Konzentration; folglich enthält die Komponente das erfindungsgemäße Polysaccharid vorzugsweise in einer höheren Konzentration als die gebrauchsfertige Dialyselösung.

Vorzugsweise ist die Konzentration an Polysaccharid in der Komponente nahe an der Sättigungskonzentration bei einer Temperatur von 5 °C um eine ausreichende Lagerstabilität bei höheren Temperaturen sicher zu stellen.

In einer bevorzugten Ausführungsform ist die Gesamtmassenkonzentration an Polysaccharid in der Komponente 0,01 g/L bis 1,0 kg/L, bevorzugter 0,1 bis 750 g/L, noch bevorzugter 1,0 bis 500 g/L, am Bevorzugtesten 10 bis 250 g/L und insbesondere 100 bis 200 g/L. In einer weiteren bevorzugten Ausführungsform ist die Gesamtmassenkonzentration an Polysaccharid in der Komponente 25 ± 24 g/L, bevorzugter 25 ± 20 g/L, noch bevorzugter 25 ± 15 g/L, am Bevorzugtesten 25 ± 10 g/L und insbesondere 25 ± 5 g/L. In einer weiteren bevorzugten Ausführungsform ist die Gesamtmassenkonzentration an Polysaccharid in der Komponente 50 ± 25 g/L, bevorzugter 50 ± 20 g/L, noch bevorzugter 50 ± 15 g/L, am Bevorzugtesten 50 ± 10 g/L und insbesondere 50 ± 5 g/L. In einer weiteren bevorzugten Ausführungsform ist die Gesamtmassenkonzentration an Polysaccharid in der Komponente 75 ± 25 g/L, bevorzugter 75 ± 20 g/L, noch bevorzugter 75 ± 15 g/L, am Bevorzugtesten 75 ± 10 g/L und insbesondere 75 ± 5 g/L. In einer weiteren bevorzugten Ausführungsform ist die Gesamtmassenkonzentration an Polysaccharid in der Komponente 100 ± 25 g/L, bevorzugter 100 ± 20 g/L, noch bevorzugter 100 ± 15 g/L, am Bevorzugtesten 100 ± 10 g/L und insbesondere 100 ± 5 g/L. In einer weiteren bevorzugten Ausführungsform ist die Gesamtmassenkonzentration an Polysaccharid in der Komponente 200 ± 25 g/L, bevorzugter 200 ± 20 g/L, noch bevorzugter 200 ± 15 g/L, am Bevorzugtesten 200 ± 10 g/L und insbesondere 200 ± 5 g/L.

In einer bevorzugten Ausführungsform enthält die zweite Komponente die Gesamtmenge an Polysaccharid und einen geeigneten Puffer, der den pH-Wert der zweiten Komponente auf über 7,0, bevorzugter auf über 7,5, noch bevorzugter auf über 8,0, am Bevorzugtesten auf über 8,5 und insbesondere auf über 9,0 einstellt. Dies kann vorzugsweise durch Hydrogencarbonat erreicht werden, die beispielsweise in Form von dissoziiertem NatriumHydrogencarbonat und oder Kalium-Hydrogencarbonat vorliegen können. In einer weiteren bevorzugten Ausführungsform ist die zweite Komponente fest und umfasst ein pulverförmiges Gemisch enthaltend wenigstens ein Polysaccharid und wenigstens einen Puffer, z.B. Natrium- und/ oder Kalium-Hydrogencarbonat.

Der Mehrkammer-Beutel ist vorzugsweise geeignet für die Herstellung einer Dialyselösung, die zur Verwendung in der Peritonealdialysebehandlung verwendet werden kann, und die folgenden Inhaltsstoffe vorzugsweise in folgenden Konzentrationen enthält:

| | |
|---|---|
| Ca^{2⊕} | 0,5 bis 5 mval/L; |
| Mg^{2⊕} | 0 bis 3,0 mval/L; |
| Cl^{Θ} | 90,5 bis 121 mval/L; |
| K^{⊕} | 0 bis 4,0 mval/L; |
| HCO₃^{Θ} | 25 bis 40 mval/L; wobei |

eine Kammer des Mehrkammer-Beutel-Systems ein erstes saures Konzentrat und eine andere Kammer ein zweites basisches Konzentrat enthält; wobei das saure Konzentrat Ca^{2⊕}-Ionen enthält und das basische Konzentrat HCO₃^{Θ}-Ionen aber keine Ca^{2⊕}-Ionen enthält; und die zwei Konzentrate nach Lösen bzw. Brechen des Trennsystems (z.B. Trennnaht) miteinander gemischt werden können; wobei das Mischen der zwei Konzentrate zur Herstellung der gebrauchsfertigen Dialyselösung führt und der pH der gebrauchsfertigen Dialyselösung 7,0 bis 7,6 ist.

Vorzugsweise enthält das basische Konzentrat wenigstens ein erfindungsgemäßes Polysaccharid und ggf. Glucose und/ oder Polyglucose, wohingegen das saure Konzentrat kein erfindungsgemäßes Polysaccharid und keine Glucose und/ oder Polyglucose enthält.

Vorzugsweise enthält das basische Konzentrat eine Menge an Hydrogencarbonat, die zu einer Hydrogencarbonat-Konzentration der gebrauchsfertigen Dialyselösung von wenigstens 20 mM führt. Vorzugsweise ist die Hydrogencarbonat-Konzentration der basischen Komponente so hoch, dass die gebrauchsfertige Dialyselösung eine Hydrogencarbonat-Konzentration von 25 mM aufweist.

Der pH-Wert des basischen, gepufferten zweiten Konzentrats wird vorzugsweise mit Salzsäure eingestellt.

Vorzugsweise werden die beiden Konzentrate in einem Volumenverhältnis von 10:1 bis 1:10 oder 8:1 bis 1:8, bevorzugter 5:1 bis 1:5 oder 3:1 bis 1:3, noch bevorzugter 2:1 bis 1:2 und insbesondere 1:1 miteinander gemischt.

Der Mehrkammer-Beutel weist vorzugsweise eine Gasbarriere-Folie auf, die verhindert, dass gasförmiges CO₂ aus dem System entweicht. Gasbarriere-Folien sind dem Fachmann bekannt.

Die Herstellung einer Dialyselösung kann so erfolgen, dass das gewünschte Mischverhältnis automatisch durch eine Dialysemaschine oder einen Peritonealdialyse-Cycler erfolgt.

Auch eine feste Zusammensetzung kann zur Herstellung der erfindungsgemäßen Dialyselösung durch das Lösen in einem definierten Volumen eines Lösungsmittels (z.B. Wasser) geeignet sein. Vorzugsweise ist die feste Zusammensetzung eine vorstehend beschriebene Komponente und somit ein Bestandteil des Kits.

Die feste Zusammensetzung enthält das Polysaccharid in einer beliebigen festen Form, z.B. als Pulver, Granulat, Pellets, etc. Das Polysaccharid kann als Lyophilisat oder sprühgetrocknet vorliegen.

Die erfindungsgemäße feste Zusammensetzung enthält vorzugsweise ein Hydrogencarbonat-Salz wie beispielsweise Natrium- oder Kalium-Hydrogencarbonat. Das Stoffmengenverhältnis von Hydrogencarbonat zu Polysaccharid in der festen Zusammensetzung ist vorzugsweise 1:100 bis 100:1, bevorzugter 1:50 bis 50:1, noch bevorzugter 1:25 bis 25:1, am Bevorzugtesten 1:10 bis 10:1 und insbesondere 1:5 bis 5:1.

Das definierte Volumen an Lösungsmittel, das zur Herstellung der erfindungsgemäßen Dialyselösung durch Lösen der festen Zusammensetzung benötigt wird ist vorzugsweise 1,0 bis 2000 1. Vorzugsweise ist das Lösungsmittel gereinigtes Wasser, sterilisiertes Wasser oder Wasser für Injektionszwecke, das ggf. einen oder mehrere der oben beschriebenen Elektrolyte, eine oder mehrere osmotisch wirksame Substanzen (z.B. wenigstens ein Polysaccharid) und/ oder einen oder mehrere der oben beschriebenen Puffer enthalten kann.

### Beispiele

### Beispiel 1

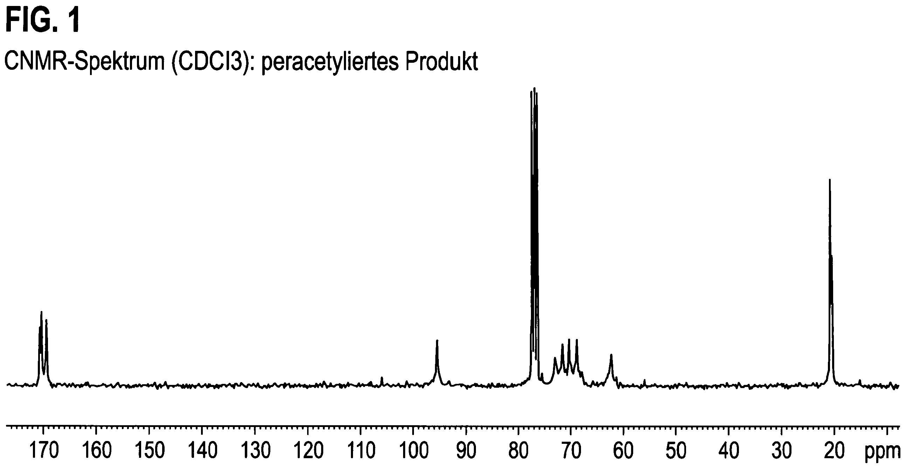

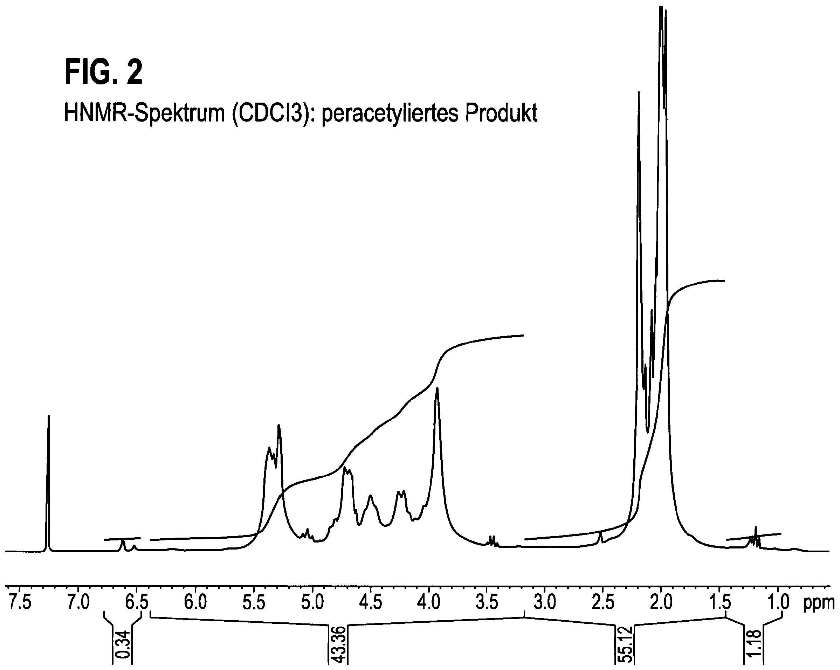

50 g abgebaute Stärke werden in 500 mL DMSO gelöst. Nach Auflösung der Stärke werden 1 Spatelspitze DMAP und 22,7 g Maleinsäureanhydrid zugegeben (0,75 mol/AGU). Das Gemisch wird für 5 Stunden bei 40 °C gerührt. Die Fällung des Produkts erfolgt in Aceton mit anschließender Filtrierung und Trocknung im Vakuum bei 40 °C. HNMR und CNMR-Spektren wurden aufgenommen (siehe Fig. 1 und Fig. 2): Substitutitonsgrad unter 0,1.

### Beispiel 2

60 g getrocknete abgebaute Stärke (0,370 mol) werden in 600 mL getrocknetem Dimethylacetamid gelöst. Nach Auflösung der Stärke werden 1 Spatelspitze DMAP, 37,2 g Triethylamin und 36,36 g Maleinsäureanhydrid zugegeben. Das Gemisch wird für 5 Stunden bei 60 °C gerührt. Die Fällung des Produkts erfolgt in Aceton mit anschließender Filtrierung und Trocknung im Vakuum bei 40 °C. HNMR und CNMR-Spektren wurden aufgenommen.

### Beispiel 3

Wie Beispiel 2, aber ohne Zusatz von Triethylamin.

Die erhaltenen erfindungsgemäßen Stärkemaleate zeigen im Vergleich zur nicht-substituierten abgebauten Stärke bei gleicher Konzentration eine erhöhte Osmolalität (experimentell bestimmt mittels Gefrierpunktserniedrigung) und einen stark erhöhten kolloidosmotischen Druck und Ultrafiltration.

### Beispiel 5

In einem vergleichenden Versuch wurde ein Füllvolumen von 10 ml eines osmotischen Agens in einer Konzentration von 5 % (m/m) in einer Versuchslösung mit 1 mmol/l Ca2+, 0,5 mmol/l Mg2+, 138 mmol/l Na+, 106 mmol/l Cl- und 35 mmol/l Lactat in einen semipermeablen Schlauch (regenerierte Cellulose, MWCO: 1000, Fa. Roth) gefüllt und bei einer Temperatur von 38°C in einem Bad der gleichen Versuchslösung für 24 Stunden unter Bewegung gelagert. Zu verschiedenen Zeitpunkten wurde die Volumenzunahme des Füllvolumens des Schlauchs ermittelt, die die osmotische Wirkung des Agens wiederspiegelt. Als osmotische Agenzien kamen 2 Stärkemaleate gemäß vorliegender Erfindung sowie die etablierten osmotischen Agenzien Glukose und Icodextrin zum Einsatz.
Die in dem Versuch verwendeten Stärkemaleate haben folgende Strukturformel: 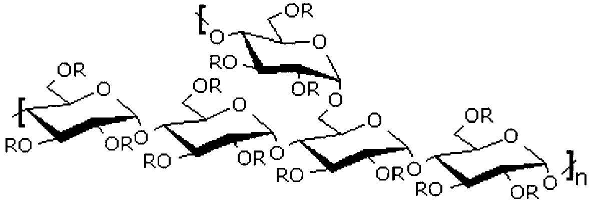 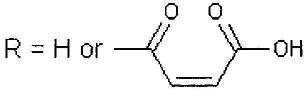 Die 2 Stärkemaleate unterscheiden sich im Substitutionsgrad (DS). Stärkemaleat 1 hat einen Substitutionsgrad von 0,1. Stärkemaleat 2 hat einen Substitutionsgrad von 0,5.

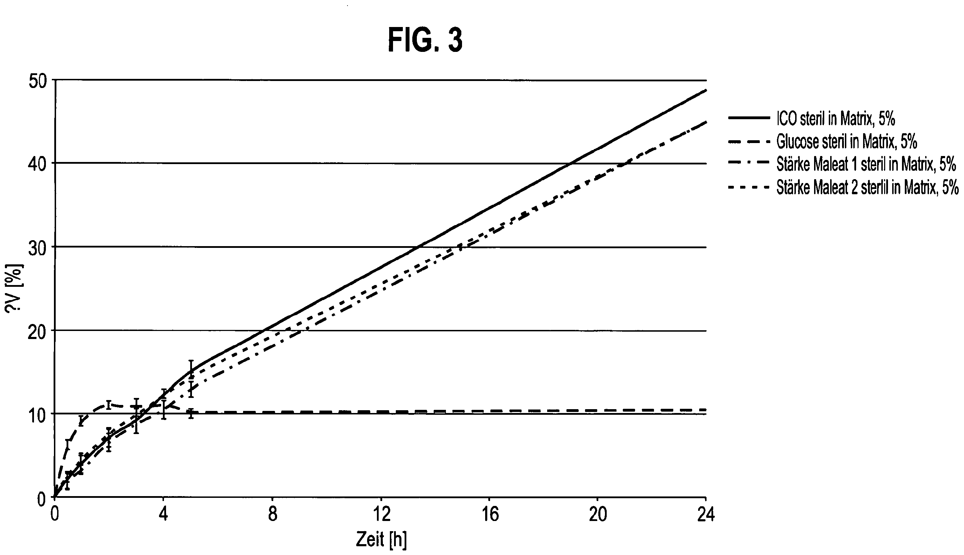

Die Ergebnisse sind in der Figur 3 als Diagramm dargestellt.

Die Stärkemaleate zeigen nach einem Zeitraum von mehr als 4 Stunden gegenüber Glukose eine deutlich stärkere osmotische Wirkung und gegenüber Icodextrin eine geringfügig geringere osmotische Wirkung.

## Patentansprüche

1. Lösung zur Verwendung in der Dialyse, enthaltend wenigstens ein Polysaccharid als Osmotikum, wobei es sich bei dem Polysaccharid um abgebaute Stärke mit einem mittleren Molekulargewicht von zwischen 2000 und 30000 g/mol handelt, dessen Glukose-Einheiten zumindest teilweise mit Maleinsäure verestert sind.

2. Lösung zur Verwendung nach Anspruch 1, wobei das Polysaccharid einen Substitutionsgrad zwischen 0,01 und 3 aufweist.

3. Lösung zur Verwendung nach einem der Ansprüche 1 bis 2, **dadurch gekennzeichnet, dass** es sich bei der Dialysebehandlung um eine Hämodialyse- oder Peritonealdialysebehandlung handelt.

## Claims

1. A solution for use in dialysis, containing at least one polysaccharide as an osmotic agent, wherein the polysaccharide is a degraded starch having an average molecular weight between 2000 and 30,000 g/mol, whose glucose units are at least in part esterified with maleic acid.

2. A solution for use according to claim 1, wherein the polysaccharide has a degree of substitution between 0.01 and 3.

3. A solution for use according to one of claims 1 to 2, **characterized in that** the dialysis treatment is a hemodialysis or peritoneal dialysis treatment.

## Revendications

1. Solution destinée à être utilisée dans la dialyse, contenant au moins un polysaccharide comme agent osmotique, dans laquelle le polysaccharide est de l'amidon dégradé doté d'un poids moléculaire moyen compris entre 2000 et 30000 g/mol, dont les unités de glucose sont estérifiées au moins partiellement avec de l'acide maléique.

2. Solution destinée à être utilisée selon la revendication 1, dans laquelle le polysaccharide présente un degré de substitution compris entre 0,01 et 3.

3. Solution destinée à être utilisée selon l'une des revendications 1 à 2, **caractérisée en ce que** le traitement de dialyse est un traitement d'hémodialyse ou de dialyse péritonéale.
